(19) Europäisches Patentamt
European Patent Office
Office européen des brevets

(11) **EP 2 150 172 B1**

(12) **EUROPÄISCHE PATENTSCHRIFT**

(45) Veröffentlichungstag und Bekanntmachung des Hinweises auf die Patenterteilung:
**09.02.2011 Patentblatt 2011/06**

(21) Anmeldenummer: **08759047.7**

(22) Anmeldetag: **05.06.2008**

(51) Int Cl.:
***A61B 5/022*** *(2006.01)*

(86) Internationale Anmeldenummer:
**PCT/EP2008/004497**

(87) Internationale Veröffentlichungsnummer:
**WO 2008/148556 (11.12.2008 Gazette 2008/50)**

(54) **VORRICHTUNG UND VERFAHREN ZUR KORREKTUR EINES GEMESSENEN BLUTDRUCKS**

DEVICE AND METHOD FOR CORRECTING A MEASURED BLOOD PRESSURE

PROCÉDÉ ET DISPOSITIF DESTINÉS À CORRIGER UNE MESURE DE PRESSION SANGUINE

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **06.06.2007 DE 102007026402**

(43) Veröffentlichungstag der Anmeldung:
**10.02.2010 Patentblatt 2010/06**

(73) Patentinhaber: **Fraunhofer-Gesellschaft zur Förderung der angewandten Forschung e.V.
80686 München (DE)**

(72) Erfinder:
• **KUSCHEL, Christian
90489 Nürnberg (DE)**

• **MÖRSDORF, Hans-Joachim
90763 Fürth (DE)**
• **ASCHENBRENNER, Stefan
90542 Eckental (DE)**

(74) Vertreter: **Schenk, Markus et al
Schoppe, Zimmermann, Stöckeler & Zinkler
Patentanwälte
Postfach 246
82043 Pullach bei München (DE)**

(56) Entgegenhaltungen:
**DE-A1- 19 757 974        US-A1- 2003 013 976
US-A1- 2005 256 411**

EP 2 150 172 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung bezieht sich auf eine Vorrichtung und auf ein Verfahren zur Korrektur eines gemessenen Blutdrucks an einem Messort und insbesondere auf eine Bestimmung der Armhöhe zur Verbesserung der Messergebnisse bei der peripheren Blutdruckmessung an einem Menschen.

Der gemessene Blutdruck eines Menschen aber auch eines Tieres hängt unter anderem von einem Messort ab. So ist aufgrund der Schwerkraft beispielsweise der Blutdruck in einem Fuß natürlicherweise höher als in einer Kopfregion - zumindest bei aufrecht stehender Position. Um derart schwerkraftbedingte Fehler zu vermeiden, erfolgt eine Blutdruckmessung typischerweise auf gleicher Höhe des Herzens. Die Blutdruckmessung kann jedoch auch an anderen Stellen des Körpers geschehen, sofern dabei der entstehende Fehler infolge der abweichenden Höhe zum Herzen ausgeglichen wird. So ist beispielsweise bei der Messung des Blutdrucks an einem Handgelenk, wie es bei handelsüblichen Geräten der Fall ist, die Höhe der Hand über dem Herzen ein wesentlicher Einflussfaktor auf den gemessenen Blutdruck.
Soll der gemessene Blutdruck von dem durch die "falsche Messhöhe" verursachten Fehler befreit werden, ist eine möglichst einfache Ermittlung der Höhe der Messanordnung über dem Herzen erforderlich. Eine Vorrichtung zur Korrektur des gemessenen Blutdrucks auf eine Bestimmung der Armhöhe mittels die Messung eines Neigungwinkels ist aus DE-A-19757974 bekannt. Der vorliegenden Erfindung liegt die Aufgabe zugrunde, eine Vorrichtung und ein Verfahren zur Korrektur eines gemessenen Blutdrucks zu schaffen, die flexibel und kostengünstig eine Höhenkorrektur während einer Blutdruckmessung durchführt.

Diese Aufgabe wird durch eine Vorrichtung gemäß Anspruch 1 und ein Verfahren gemäß Anspruch 13 gelöst.

Der vorliegenden Erfindung liegt die Erkenntnis zugrunde, dass eine Vorrichtung zur Ermittlung einer Höhe eines Messgerätes im Vergleich zu einem Referenzpunkt dadurch geschaffen werden kann, dass das Messgerät einen Sender aufweist oder ein Sender sich möglichst in der Nähe des Messgeräts befindet, und der ein Signal, beispielsweise in Form einer elektromagnetischen Welle mit gleichbleibender Frequenz, aussendet und das Signal von zumindest drei Empfängern empfangen wird. Die drei Empfänger senden ein Empfangssignal an einer Auswerteeinheit, so dass in der Auswerteeinheit ein Laufzeit- oder ein Phasenunterschied der empfangenen Signale an zwei der drei Empfänger ermittelt werden kann. Der Laufzeit- oder Phasenunterschied bestimmt seinerseits einen Wegunterschied zwischen Wegen, den das Signal zu dem ersten der zwei Sender und zu dem zweiten der zwei Sender zurückgelegt hat. In ähnlicher Art und Weise kann ein weiterer Wegunterschied während der Ausbreitung zu einem dritten der drei Sender und dem zweiten oder dem ersten der drei Sender ermittelt werden.

Die Empfangssignale der drei Empfänger können dabei beispielsweise elektrische Wechselsignale sein, deren Phasen in einer festen Beziehung zu den Phasen der durch die Empfänger empfangenen Wellen (oder Signale) stehen. In diesem Fall kann mittels eines Phasendiskriminators Phasenunterschiede der elektrischen Wechselsignale in einfache Weise bestimmt werden und die Auswerteeinheit kann daraus mittels der Frequenz und Ausbreitungsgeschwindigkeit eine Weglängendifferenz oder Wegunterschiede ermitteln. Es ist ebenfalls möglich, dass die Phaseninformationen der empfangenen Welle auf andere Weise (z.B. digitale) erfasst und an die Auswerteeinheit weitergegeben werden.

Sofern die drei Empfänger sich am Körper in verschiedenen Höhen, vorzugsweise entlang einer senkrechten Linie (d. h. möglichst im Lot), befinden, kann mit Hilfe der Wegunterschiede (d.h. der Laufzeit- oder Phasenunterschiede) die relative Höhe des Senders bezüglich den drei Empfängern bestimmt werden. Unter der Annahme, dass die drei Empfänger sich in einem vorbestimmten oder bekannten Abstand zu dem Herzen befinden, kann daraus schließlich eine Höhenabweichung des Senders im Vergleich zum Herzen ermittelt werden. Die ermittelte Höhenabweichung kann schließlich genutzt werden, um einen Korrekturwert zu ermitteln, um den die Blutdruckmessung korrigiert wird. Sofern sich der Sender, gemäß Ausführungsbeispielen der vorliegenden Erfindung, möglichst Nahe am Messgerät befindet, kann eine möglichst genaue Bestimmung der Höhenabweichung geschehen.

Somit beschreibt die vorliegende Erfindung eine Vorrichtung zur Korrektur eines gemessenen Blutdrucks an einem Messort an einem Lebewesen (beispielsweise einem Menschen), wobei die Vorrichtung einen Sender, zumindest drei Empfänger und eine Auswerteeinheit aufweist. Der Sender sendet ein Signal von einer Nähe des Messortes aus und die drei Empfänger empfangen das Signal, wobei die drei Empfänger an Orten in unterschiedlicher Höhe des Lebewesens anbringbar sind. Die Auswerteeinheit ist ausgelegt, um eine Korrektur des gemessenen Blutdrucks auf Basis von Laufzeit- oder Phasenunterschieden der an den drei Empfängern ankommenden Signale durchzuführen.

Bei Ausführungsbeispielen weist die Messanordnung einen Sender auf, der an einem Handgelenk befestigt wird, und drei Empfänger, die am Torso beispielsweise eines Menschen - etwa an Schulter und Taille - angebracht werden. Der Sender sendet eine elektromagnetische Welle aus, die von den drei Empfängern erfasst wird. Aufgrund der relativen Lage des Senders zu den drei Empfängern ergeben sich in den Empfängern Phasenunterschiede, die sich in Phasendifferenzen äußern. Diese Phasendifferenzen können von Phasendiskriminatoren erkannt und gemessen. Mittels der bekannten Ausbreitungsgeschwindigkeit (Lichtgeschwindigkeit für eine elektromagnetische Welle) werden die Phasendifferenzen in Bruchteilen von Wellenlängen umgerechnet, um die sich die Entfernungsdifferenzen voneinander unterscheiden.

Die Frequenz der elektromagnetischen Welle kann dabei derart gewählt werden, dass in einem zu erfassenden Bereich (beispielsweise einer doppelten Armlänge) nicht mehrere komplette Wellenzüge liegen, da ansonsten der Phasenwinkel bzw. die Phasenwinkeldifferenz nicht eindeutig bestimmt werden kann und somit ebenfalls die Lage bzw. Höhenabweichung nicht eindeutig bestimmbar ist. Die doppelte Armlänge bezieht sich dabei beispielsweise auf die beiden Extremwerte, dass das Blutdruckmessgerät oder der Sender sich am Handgelenk befindet und sich die Hand zum einen senkrecht nach unten und zum anderen senkrecht nach oben ausgestreckt sein kann. Um eine eindeutige Messung sicherzustellen, ist deshalb eine untere Grenze für die verwendete Wellenlänge (=minimale Wellenlänge) der elektromagnetischen Welle zu beachten.

Andererseits sollte die Wellenlänge der verwendeten elektromagnetischen Wellenlänge jedoch nicht zu groß gewählt sein (oder die Frequenz sollte nicht zu klein gewählt sein), damit sich der Phasenwinkel an zumindest zwei der drei Empfänger sich soweit voneinander unterscheidet, dass eine eindeutige Detektion der Phasendifferenz möglich ist. Somit liegt die Wellenlänger unterhalb einer maximalen Wellenlänge. Bei sehr großen Wellenlängen wird sich nämlich der Amplitudenwert der empfangenen elektromagnetischen Welle an den drei Empfängern nur marginal unterscheiden, so dass dieser marginale Unterschied unterhalb einer Messschwelle (Messtoleranz, z.B. sich ein Amplitudenwert um weniger als 5 % ändert) liegen kann. Da eine möglichst lotrechte Positionierung der drei Empfänger entlang des Körpers bereits eine Fehlerquelle darstellt, ist es vorteilhaft, den Wellenlängenbereich der elektromagnetischen Welle derart zu wählen, dass eine leichte Detektion der Phasendifferenzen Empfängern möglicht wird. Mit anderen Worten gesagt, dass der erfasste Phasenunterschied (bzw. der entsprechende Längenunterschied) sich deutlich von dem Fehler, infolge von Abweichungen von einer ideal-lotrechter Ausrichtung der Empfänger, unterscheidet (beispielsweise um mehr als 20 %). Beispielsweise kann die Wellenlänge in einem Bereich zwischen 10 cm und 200 cm oder in einem Bereich zwischen 40 und 120 cm liegen.

Bei weiteren Ausführungsbeispielen ist der Sender direkt in das Blutdruckmessgerät integriert. Ferner ist es möglich, die Entfernung zwischen den drei Empfängern derart zu bestimmen, dass der Sender zwecks einer Eichung in die Nähe eines der drei Sender gebracht wird, so dass die Phasendifferenzen der empfangenen Signale an den anderen der drei Sender ein direktes Maß für die Entfernung (oder den Höhenunterschied) der anderen Sender zu dem einen der drei Sender ist. Alternativ kann die Entfernung der drei Empfänger auch mit anderen konventionellen Methoden gemessen werden.

Bei weiteren Ausführungsbeispielen weist die Vorrichtung noch weitere Empfänger auf, so dass dann über eine Mittelung eine Erhöhung der Messgenauigkeit erzielt werden kann. Da die Höhenbestimmung mit vier Empfängern bereits überbestimmt ist, kann der vierte und/oder jeder weitere Empfänger für eine Bestimmung einer Fehlerrate herangezogen werden. Bei vier oder mehr Empfängern kann die bestimmte Fehlerrate auch dazu genutzt werden, um eine Optimierung hinsichtlich der Wellenlänge der elektromagnetischen Welle durchzuführen, um somit in einen optimalen Wellenlängenbereich, der beispielsweise eine eindeutige Messung bei minimalen Fehler erlaubt, durch einen Wechsel der Wellenlänge des Senders zu erreichen. Ferner ist es möglich bei Verwendung von gepulsten Signalen Laufzeitmessungen der Pulse durchzuführen und somit Wegedifferenzen direkt aus Laufzeitunterschieden zu ermitteln. Hierfür können sowohl einfache Wegstrecken als auch doppelte Wegstrecken (Hin und Zurück) über an den Empfängern reflektierte Signale verwendet werden.

Die vorliegende Erfindung ist dahin gehend vorteilhaft, da sie unter Verwendung einfacher Standardkomponenten eine einfache und effektive und außerdem kostengünstige Möglichkeit bietet, eine Höhenkorrektur einer Blutdruckmessung durchzuführen. Die erfindungsgemäße Vorrichtung bzw. das erfindungsgemäße Verfahren ist flexibel einsetzbar und selbst bei einer momentanen Änderung der relativen Höhe (im Vergleich zum Herzen beispielsweise) kann eine dynamische Anpassung des gemessenen Blutdruckmesswertes durchgeführt werden.

Bevorzugte Ausführungsbeispiele der vorliegenden Erfindung werden nachfolgend Bezug nehmend auf die beiliegenden Zeichnungen näher erläutert. Es zeigen:

Fig. 1    eine Prinzipdarstellung eines Ausführungsbei- spiels der vorliegenden Erfindung;

Fig. 2    eine Darstellung der geometrischen Größen zur Bestimmung einer Höhenabweichung;

Fig. 3a    eine Darstellung zur Optimierung einer Wellenlän- ge der elektromagnetischen Welle; und

Fig. 3b    eine Darstellung einer ungünstige gewählten Wellenlänge der elektromagnetischen Welle.

Bevor im Folgenden die vorliegende Erfindung anhand der Zeichnungen näher erläutert wird, wird darauf hingewiesen, dass gleiche Elemente in den Figuren mit den gleichen oder ähnlichen Bezugszeichen versehen sind und dass eine wiederholte Beschreibung dieser Elemente weggelassen wird.

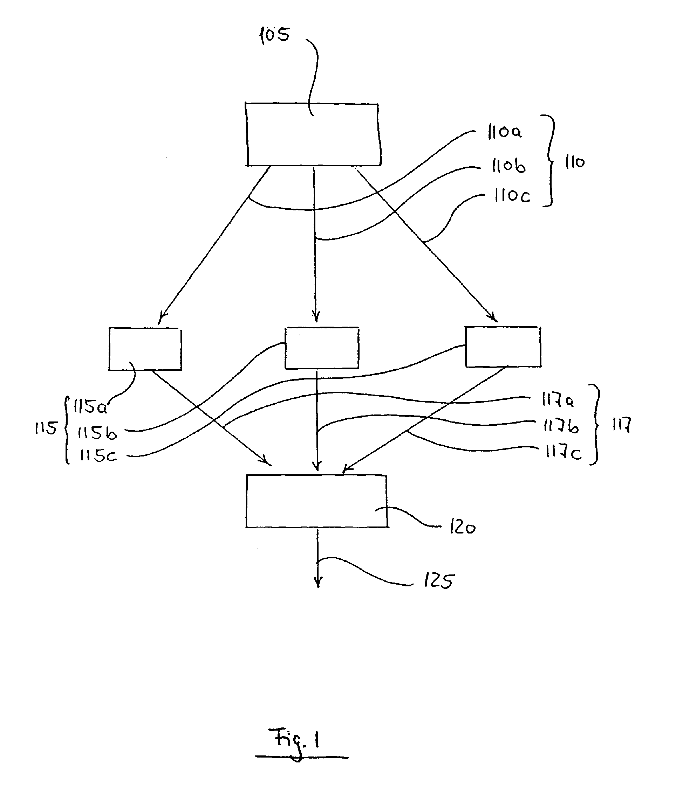

Fig. 1 zeigt eine Prinzipdarstellung eines Ausführungsbeispiels der vorliegenden Erfindung. Ein Sender 105 sendet eine elektromagnetische Welle 110 mit einer vorbestimmten Wellenlänge aus. Ein erster Teil der elektromagnetischen Welle 110a erreicht einen ersten Empfänger 115a, ein zweiter Teil der elektromagnetischen Welle 110b erreicht einen zweiten Empfänger 115b und ein dritter Teil der elektromagnetischen Welle 110c erreicht einen dritten Empfänger 115c. Der erste Empfänger 115a erfasst den ersten Teil der elektromagnetischen Welle 110a und übermittelt ein erstes Empfangssignal 117a an eine Auswerteeinheit 120. Der zweite Empfänger 115b erfasst den zweiten Teil der elektromagnetischen Welle 110b und übermittelt ein zweites Empfangssignal 117b an die Auswerteeinheit 120. Der dritte Empfänger 115c erfasst den dritten Teil der elektromagnetischen Welle 110c und übermittelt ein drittes Empfangssignal 117c an die Auswerteeinheit 120. Das erste, zweite und dritte Empfangssignal 117a, 117b und 117c enthalten beispielsweise Informationen über die Phase der elektromagnetischen Welle 110 bzw. die Phase des ersten Teils der elektromagnetischen Welle 110a zum Zeitpunkt des Empfangs durch den ersten Empfänger 115a. In ähnlicher Weise enthält das zweite Empfangssignal 117b die Phaseninformationen des zweiten Teils der elektromagnetischen Welle 110b und das dritte Empfangssignal 117c enthält Phaseninformationen bezüglich des dritten Teils der elektromagnetischen Welle 110c (z.B. einen Phasenwert jeweils zum Zeitpunkt des Empfangs). Sofern der Sender gepulste Signale verwendet, können alternativ die drei Empfangssignale 117a, 117b und 117c auch Informationen über den Zeitpunkt des Empfangs der gepulsten Signale durch den ersten, zweiten und dritten Empfänger 115a, 115b und 115c enthalten.

Die Auswerteeinheit 120 vergleicht das erste Empfangssignal 117a, das zweite Empfangssignal 117b und das dritte Empfangssignal 117c. Beispielsweise kann die Auswerteeinheit 120 eine Phasendifferenz des ersten Teils der elektromagnetischen Welle 110a im Vergleich zum zweiten Teil der elektromagnetischen Welle 110b zum Zeitpunkt des Empfangs des ersten Teils der elektromagnetischen Welle 110a durch den ersten Empfänger 115a und des zweiten Teils der elektromagnetischen Welle 110b durch den zweiten Empfänger 115b ermitteln. Die Auswerteeinheit 120 kann aus dieser Phasendifferenz eine Längendifferenz zwischen den Längen, die der erste Teil oder der zweite Teil der elektromagnetischen Welle 110a oder 110b zurückgelegt haben, ermitteln. In ähnlicher Weise kann die Auswerteeinheit 120 eine Phasendifferenz zwischen dem zweiten Teil der elektromagnetischen Welle 110b und dem dritten Teil der elektromagnetischen Welle 110c zum Zeitpunkt des Empfangs durch den zweiten Empfänger 115b und des dritten Empfängers 115c feststellen und daraus wiederum eine Längendifferenz ermitteln. Alternativ können bei der Verwendung eines gepulsten Senders die Zeitdifferenzen beim Empfang in Längendifferenzen umgerechnet werden.

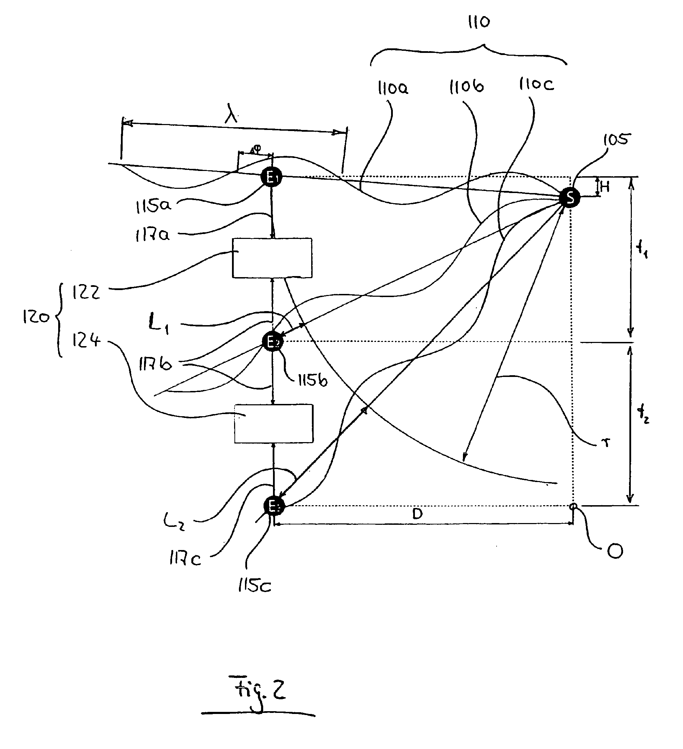

Aus den daraus ermittelten Längendifferenzen (nähere Details werden in der Fig. 2 beschrieben) und aus den Höhendifferenzen zwischen den ersten, zweiten und dritten Empfängern 115a, 115b und 115c kann die Auswerteeinheit eine Höhenabweichung 125 bestimmen, um den sich der Sender 105 relativ zu einem der drei Empfänger 115a, 115b und 115c befindet. Die Höhenabweichung 125 kann dann dazu genutzt werden, um einen gemessenen Blutdruck entsprechend zu korrigieren.

Fig. 2 zeigt eine Darstellung der geometrischen Größen zur Bestimmung der Höhenabweichung 125, die im Folgenden mit H bezeichnet wird. Die drei Empfänger 115a, 115b und 115c sind in dieser Darstellung lotrecht übereinander angeordnet, wobei der dritte Empfänger 115c sich auf einer Grundlinie im Abstand D von einem Koordinatenursprung O befindet. In einem Abstand $t_2$ befindet sich der zweite Empfänger 115b senkrecht (bzw. lotrecht) über dem dritten Empfänger 115c und der erste Empfänger 115a befindet sich in einem Abstand $t_1$ senkrecht über dem zweiten Empfänger 115b. Der Koordinatenursprung O ist so gewählt, dass der Sender 105 sich senkrecht bzw. lotrecht über dem Koordinatenursprung O angeordnet ist. Der Sender 105 ist in der Fig. 2 ebenfalls mit S bezeichnet, der erste Empfänger 115a ist mit $E_1$ und der zweite Empfänger 115b ist mit $E_2$ und der dritte Empfänger 115c ist mit $E_3$ gekennzeichnet. Der erste Empfänger 115a befindet sich in einem radialen Abstand r von dem Sender 105, der zweite Empfänger 115b befindet sich in einem zweiten radialen Abstand $r + L_1$ von dem Sender 105 und der dritte Empfänger 115c befindet sich in einem dritten radialem Abstand $r + L_2$ von dem Sender 105.

Die elektromagnetische Welle 110, die von dem Sender 105 gesendet wird, weist eine Wellenlänge λ auf und zum Zeitpunkt des Empfangs durch den ersten Empfänger 115a einen Phasenwert von $\pi - \Delta\varphi$ auf (Winkel werden im Folgenden im Bogenmaß angegeben). Da der Abstand des Senders 105 zu dem zweiten Empfänger 115b um die erste Länge $L_1$ größer ist als der Abstand des ersten Empfänger 115a zu dem Sender 105, hat sich der Phasenwert der empfangenen elektromagnetischen Welle 110b beim zweiten Empfänger 115b entsprechend geändert. In ähnlicher Weise ist der Phasenwert des dritten Teils der elektromagnetischen Welle 110c zum Zeitpunkt des Empfangs durch den dritten Empfänger 115c infolge der um die dritte Weglänge $L_2$ vergrößerten Abstandes des dritten Empfängers 115c von dem Sender 105 entsprechend verschoben.

Durch Anwendung trigonometrischer Gesetzmäßigkeiten lässt sich die Lage des Punktes S (Sender 105) relativ zu den Punkten $E_1$ (erster Empfänger 115a), $E_2$ (zweiter Empfänger 115b) und $E_3$ (dritter Empfanger 115c) angeben und damit lässt sich die (relative) Hohe H des Punktes S (beispielsweise das Handgelenk) bezüglich $E_1$ (beispielsweise der Schulter) ermitteln. Die Lagebestimmung des Punktes S erfolgt in $R^2$ (d. h. innerhalb einer Ebene) auf zwei mögliche Lagen genau - links und rechts oder spiegelsymmetrisch bzgl. der lotrechten Gerade zwischen $E_1$ und $E_3$. Vom Raum $R^3$ aus betrachtet, bedeutet dies, dass die Lagebestimmung auf einer Kreisbahn mit einem Mittelpunkt auf der Geraden $E_1E_2$ genau erfolgt. Die Position entlang es Kreises bleibt dabei allerdings unbestimmt - nur die Lage des Kreises in $R^3$ ist bestimmt. Da für die anvisierte Anwendung jedoch lediglich die Höhe H von Interesse ist, reicht die erzielbare Auflösung der Lage aus.

Im Detail werden zur Bestimmung der relativen Höhe H (z. B. des Handgelenks über oder unter der Höhe von $E_1$) ein Gleichungssystem aus drei Gleichungen und drei Unbekannten gelöst. Wie aus Fig. 2 zu entnehmen ist, werden dabei folgende Variablen benutzt:

$\overline{E_1 E_2}$ sei $t_1$,
$\overline{E_2 E_3}$ sei $t_2$,
$\overline{SE_1}$ sei r und
D sei der lotrechte Abstand von S zur Geraden $E_1 E_2$

Mit dieser Definition ergeben sich folgende Beziehungen:

$$r^2 = H^2 + D^2 \,, \tag{1}$$

$$(r + L_1)^2 = (t_1 - H)^2 + D^2 \,, \tag{2}$$

$$(r + L_2)^2 = (t_2 + t_1 - H)^2 + D^2 \,. \tag{3}$$

Durch Einsetzen von $D^2 = r^2 - H^2$ aus (1) in (2) und (3) erhält man folgende Gleichungen:

$$0 = t_1^2 - 2 \cdot H \cdot t_1 - 2 \cdot r \cdot L_1 - L_1^2 \,, \tag{4}$$

$$0 = (t_1 + t_2)^2 - 2 \cdot H \cdot (t_1 + t_2) - 2 \cdot r \cdot L_2 - L_2^2 \,. \tag{5}$$

Auflösen von (5) nach r und Einsetzen in (4) ergibt:

$$H = \frac{L_1(t_1 + t_2)^2 - L_2(t_1^2 - L_1^2) - L_1 \cdot L_2^2}{2 \cdot ((t_1 + t_2) \cdot L_1 - t_1 \cdot L_2)} \tag{6}$$

Der erste Empfänger 115a und der zweite Empfänger 115b übermitteln bei diesem Ausführungsbeispiel die Empfangssignale 117a, 117b an die Auswerteeinheit 120, die einen ersten Phasendiskriminator 122 aufweist, der eine erste Phasendifferenz $\Delta\varphi_1$ zwischen der Phase des ersten Teils der elektromagnetischen Welle 110a, die vom ersten Empfänger 115a erfasst wird, und der Phase des zweiten Teils der elektromagnetischen Welle 110b, die von dem zweiten Empfänger 115b erfasst wird, ermittelt. In gleicher Weise sendet der zweite Empfänger 115b und der dritte Empfänger 115c die Empfangssignale 117b, 117c zu einem zweiten Phasendiskriminator 124, der eine zweite Phasendifferenz $\Delta\varphi_2$ zwischen den Phasen der erfassten elektromagnetischen Welle am zweiten Empfänger 115b und der erfassten elektromagnetischen Welle am dritten Empfänger 115c feststellt. Die erste Phasendifferenz $\Delta\varphi_1$, die am ersten Phasendiskriminator ermittelt wird, und die zweite Phasendifferenz $\Delta\varphi_2$, die am zweiten Phasendiskriminator 124 ermittelt wird, können durch die folgenden Beziehungen in die erste Längendifferenz $L_1$ und die zweite Längendifferenz $L_2$ umgerechnet werden:

$$L_1 = \frac{\Delta\varphi_1}{2\pi} \lambda \,, \tag{7}$$

$$L_2 = \frac{\Delta\varphi_2}{2\pi}\lambda \qquad\qquad (8)$$

wobei wie gesagt eine Winkelmessung im Bogenmaß vorgenommen wird, d. h. die Phase $\varphi$ ist $2\pi$-periodisch.

Somit werden in der Anordnung, wie sie in Fig. 2 dargestellt ist, zwei Messungen durchgeführt, eine Messung zur Bestimmung der ersten Phasendifferenz $\Delta\varphi_1$, die wiederum die erste Längendifferenz $L_1$ festlegt, und eine zweite Messung, die die zweite Phasendifferenz $\Delta\varphi_2$, die wiederum die zweite Längendifferenz $L_2$ bestimmt. Da die relative Position der Empfänger $E_i$ und somit die Größen ti und $t_2$ bekannt sei, beispielsweise kann ein bestimmter Empfänger (z.B. der zweite Empfänger $E_2$) in der Nähe des Herzens angebracht sein, ist es somit möglich die relative Position des Punktes S zu dem bestimmten Empfänger zu bestimmen.

Bei einem optionalen Hinzufügen weiterer Empfänger und damit verbunden dem Durchführen weiterer Messungen, würde zusätzlich zu den Gleichungen (1) bis (3) noch eine vierte Gleichung hinzukommen und somit wäre das Gleichungssystem überbestimmt (vier Gleichungen für drei unbekannte Größen D, r und H; $t_i$ sind als bekannt vorausgesetzt oder werden ausgemessen) - die weitere Messung könnte jedoch als Kontrollmessung dienen, um damit eine Fehlerrate festzustellen, beispielsweise infolge nicht ideal senkrecht oder lotrecht ausgerichteter Empfänger $E_i$ (i zählt die Anzahl der Empfänger, z.B. i = 1, 2, 3, 4) oder aber infolge einer ungünstig gewählten Wellenlänge $\lambda$ des Senders 105. Das Bestimmen der Fehlerrate kann dabei derart geschehen, dass verschiedene drei der vier (oder mehr) Empfänger ausgewählt werden, um verschiedene Höhenabweichungen $H_i$ zu ermitteln, so dass die Streuung (z.B. ausgedrückt durch die Standardabweichung) ein Maß für die Fehlerrate darstellt. Somit wäre eine Optimierung sowohl der geometrischen Anordnung (Ausrichten der Empfänger) als auch der gewählten Wellenlänge $\lambda$ möglich.

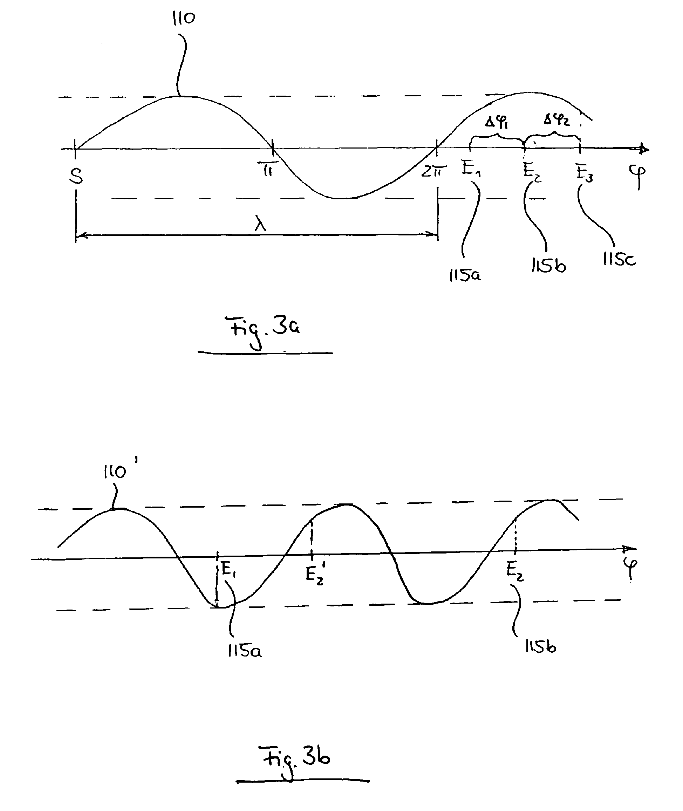

Fig. 3a zeigt eine Darstellung, bei der die drei Empfänger, der erste Empfänger $E_1$, der zweite Empfänger $E_2$ und der dritte Empfänger $E_3$, entlang einer Richtung dargestellt sind, so dass sich die unterschiedliche Entfernung zu dem Punkt S in unterschiedlichen Phasenwerten $\varphi$ für die elektromagnetische Welle 110, die von dem Sender S ausgesendet wird, zeigt. Eine Phase $\varphi = E_1$ entspricht dabei einem ersten Phasenwert, den die elektromagnetische Welle 110 bei dem Empfang durch den ersten Empfänger 115a aufweist, und die Phase $\varphi = E_2$ entspricht einem zweiten Phasenwert, den die elektromagnetische Welle 110 zu dem Zeitpunkt des Empfangs durch den zweiten Empfänger 115b aufweist, und die Phase $\varphi = E_3$ entspricht einem dritten Phasenwert, den die elektromagnetische Welle 110 zu dem Zeitpunkt des Empfangs durch den dritten Empfänger 115c aufweist. Demzufolge entspricht die erste Phasendifferenz $\Delta\varphi = E_2 - E_1$ gemäß Gleichung (7) der ersten Längendifferenz $L_1$. In gleicher Weise entspricht die zweite Phasendifferenz $\Delta\varphi_2 = E_3 - E_2$ der zweiten Längendifferenz $L_2$, gemäß obiger Gleichung (7).

Wie in der Fig. 3a ersichtlich, schwankt die elektromagnetische Welle 110 zwischen zwei Maximalwerten, die durch die gestrichelte Linie dargestellt sind und die Wellenlänge ist dabei derart gewählt, dass sich die drei Empfänger ($E_1$, $E_2$, $E_3$) innerhalb einer Periode der Welle 110 befinden und sich die Amplitude der elektromagnetischen Welle 110 zwischen dem ersten Empfänger 115a und dem dritten Empfänger 115c deutlich ändert. Die Wahl der Wellenlänge $\lambda$ der elektromagnetischen Welle 110, wie sie in der Fig. 3a gezeigt ist, entspricht somit den oben erwähnten Kriterien, so dass zwischen den Empfängern nicht mehrere Wellenperioden liegen können - somit ist die Wellenlänge $\lambda$ sowohl oberhalb der minimalen als auch unterhalb der maximalen Wellenlänge.

Im Gegensatz dazu ist in Fig. 3b eine weitere Welle 110' gezeigt, die eine deutlich kürzere Wellenlänge aufweist (im Vergleich zum Abstand der Empfänger $E_1$ und $E_2$), so dass in diesem Fall sich zwischen dem ersten Empfänger 115a und dem zweiten Empfänger 115b eine vollständige Periode der weiteren Welle 110' befindet. Die Auswerteeinheit, die die Phasendifferenz der weiteren Welle 110' am Punkt $E_1$ zu dem Punkt $E_2$ untersucht oder bestimmt, kann dabei nicht zwischen dem Punkt $E_2$ und dem Punkt $E_2$' unterscheiden. Demzufolge ist eine eindeutige Entfernungsbestimmung der Länge $L_1$ basierend auf dieser Phasendifferenz nicht möglich. Das Gleiche wäre der Fall, wenn die Wellenlänge $\lambda$ derart groß gewählt ist, dass sich die Amplitude zwischen dem Punkt $E_1$ und dem Punkt $E_2$ nur marginal ändert, so dass innerhalb einer Fehlertoleranz keine Längenbestimmung für die Längendifferenz $L_1$ möglich ist.

Die Bestimmung der Phasenunterschiede $\Delta\varphi_1$ bzw. $\Delta\varphi_2$ entspricht damit der Bestimmung von Laufzeitunterschieden der elektromagnetischen Welle von dem Sender 105 zu dem Empfänger 115a, 115b und 115c und könnte alternativ ebenso über eine Zeitmessung, z.B. unter Verwendung der oben erwähnten gepulsten Signale, erfolgen. Vorteilhaft an dem Ausführungsbeispiel aus Fig. 2, ist jedoch, dass eine Zeitsynchronisation nicht erforderlich ist und der Sender 105 kontinuierlich eine elektromagnetische Welle 110 senden kann.

**Patentansprüche**

1. Vorrichtung zur Höhenkorrektur eines gemessenen Blutdrucks an einem Messort eines Lebewesens, mit folgenden Merkmalen:

einem Sender (105) zum Aussenden eines Signals (110) von einer Nähe eines Messortes aus;
zumindest drei Empfängern (115) zum Empfangen des Signals (110), wobei die Empfänger (115) an Orten in unterschiedlicher Höhe des Lebewesens anbringbar sind; und

eine Auswerteeinheit (120) zum Korrigieren des gemessenen Blutdrucks auf der Basis von Laufzeit- oder Phasenunterschieden der an den zumindest drei Empfängern (115) empfangenen Signale.

2. Vorrichtung gemäß Anspruch 1, bei der die Auswerteeinheit (120) ausgelegt ist, aus den Laufzeit- oder Phasenunterschieden einen Höhenunterschied (125) des Senders (105) zu einem ersten Empfänger (115a) zu ermitteln.

3. Vorrichtung gemäß Anspruch 1 oder Anspruch 2, bei der das Signal eine elektromagnetische Welle (110) mit vorbestimmter Wellenlänge ($\lambda$) aufweist und bei dem die zumindest drei Empfänger (115) ausgelegt sind, Empfangssignale (117) an die Auswerteeinheit (120) zu übermitteln.

4. Vorrichtung gemäß einem Anspruch 3, bei der die Empfänger (115) Phasen der empfangenen elektromagnetischen Welle (110) erfassen und die Empfangssignale (117) Informationen über empfangene Phasenwerte aufweisen und bei der die Auswerteeinheit einen ersten Phasendiskriminator (122) zum Ermitteln einer erste Phasendifferenz $\Delta\varphi_1$ zwischen einem zweiten empfangenen Phasenwert und einem ersten empfangenen Phasenwert aufweist; und einen zweiten Phasendiskriminator (124) zum Ermitteln einer zweiten Phasendifferenz $\Delta\varphi_2$ zwischen einem dritten empfangenen Phasenwert und dem zweiten empfangenen Phasenwert aufweist.

5. Vorrichtung gemäß einem der Anspruch 3 oder Anspruch 4, bei der die vorbestimmte Wellenlänge ($\lambda$) mindestens der doppelten Armlänge eines Menschen entspricht.

6. Vorrichtung gemäß einem der Ansprüche 3 bis 5, bei der die vorbestimmte Wellenlänge ($\lambda$) unterhalb einer maximalen Wellenlänge liegt und die maximale Wellenlänge **dadurch** gegeben ist, dass sich ein Amplitudenwert der elektromagnetischen Welle (110) zwischen den zumindest drei Empfängern (115) um weniger als 5 % ändert.

7. Vorrichtung gemäß einem der Ansprüche 3 bis 6, bei der die vorbestimmte Wellenlänge ($\lambda$) in einem Bereich zwischen 10 cm und 200 cm oder in einem Bereich zwischen 40 und 120 cm liegt.

8. Vorrichtung gemäß einem der vorhergehenden Ansprüche, bei der die Auswerteeinheit (120) ausgelegt ist, einen relativen Höhenunterschied ($t_1$, $t_2$) zwischen den zumindest drei Empfängern **dadurch** zu bestimmen, dass der Sender (105) in die Nähe eines der drei Empfänger (115) positionierbar ist und auf der Basis von Laufzeit- oder Phasenunterschieden die relativen Höhenunterschiede ($t_1$, $t_2$) der drei Empfänger (115) bestimmbar ist.

9. Vorrichtung gemäß einem der vorhergehenden Ansprüche, bei der der Sender (105) an einem Blutdruckmessgerät angebracht ist.

10. Vorrichtung gemäß einem der vorhergehenden Ansprüche, bei der der Sender (105) an einem Handgelenk anbringbar ist.

11. Vorrichtung gemäß einem der vorhergehenden Ansprüche, die einen ersten Empfänger (115a), einen zweiten Empfänger (115b) und einen dritten Empfänger (115c) aufweist und der erste, zweite und dritte Empfänger (115a, 115b, 115c) am menschlichen Körper anbringbar sind, so dass der erste Empfänge (115a) in Höhe einer Schulter, der zweite Empfänger (115b) in Höhe des Herzens und der dritte Empfänger (115c) in Höhe der Taille anbringbar ist.

12. Vorrichtung gemäß einem der vorhergehenden Ansprüche, die ferner einen vierten Empfänger aufweist und der vierte Empfänger ausgelegt ist zum Empfangen des Signals (110) und die Auswerteeinheit (120) ausgelegt ist, das empfangene Signal des vierten Empfängers als eine Kontrollmessung zu nutzen, um eine Fehlerrate zu bestimmen.

13. Verfahren zur Höhenkorrektur eines gemessenen Blutdrucks an einem Messort eines Lebewesens, mit folgenden Schritten:

Senden eines Signals (110) durch einen Sender (105) von einer Nähe eines Messortes aus;
Empfangen des Signals (110) durch zumindest drei Empfänger (115), wobei die zumindest drei Empfänger (115) an Orten in unterschiedlicher Höhe des Lebewesens angebracht werden; und
Korrigieren des gemessenen Blutdrucks auf der Basis von Laufzeit- oder Phasenunterschieden des an den zumindest drei Empfängern (115) empfangenen Signals.

**14.** Verfahren gemäß Anspruch 13, das ferner ein Messen von relativen Höhenunterschieden ($t_1$, $t_2$) umfasst.

**15.** Verfahren gemäß Anspruch 13, das ferner ein Positionieren des Senders (105) an einem zweiten Empfänger (115b) und ein Ermitteln von relativen Höhenunterschieden ($t_1$, $t_2$) aus den Laufzeit- oder Phasenunterschieden umfasst.

**Claims**

**1.** An apparatus for performing a height correction of a measured blood pressure at a measuring position of a living being, comprising:

a transmitter (105) for sending out a signal (110) from near a measuring position;
at least three receivers (115) for receiving the signal (110), said receivers (115) being mountable at locations at different heights of the living being; and
an evaluation unit (120) for correcting the measured blood pressure on the basis of runtime or phase differences of the signals received at the at least three receivers (115).

**2.** Apparatus as claimed in claim 1, wherein the evaluation unit (120) is configured to determine a difference in height (125) of the transmitter (105) relative to a first receiver (115a) from the runtime or phase differences.

**3.** Apparatus as claimed in claim 1 or in claim 2, wherein the signal has an electromagnetic wave (110) with a predetermined wavelength ($\lambda$), and wherein the at least three receivers (115) are configured to transmit receive signals (117) to the evaluation unit (120).

**4.** Apparatus as claimed in claim 3, wherein the receivers (115) detect phases of the received electromagnetic wave (110), and the receive signals (117) comprise information about received phase values, and wherein the evaluation unit has a first phase discriminator (122) for determining a first phase difference $\Delta\varphi_1$ between a second received phase value and a first received phase value; and
a second phase discriminator (124) for determining a second phase difference $\Delta\varphi_2$ between a third received phase value and the second received phase value.

**5.** Apparatus as claimed in one of claim 3 or claim 4, wherein the predetermined wavelength ($\lambda$) corresponds to at least double the arm length of a human being.

**6.** Apparatus as claimed in any of claims 3 to 5, wherein the predetermined wavelength ($\lambda$) is below a maximum wavelength, and the maximum wavelength is defined by the fact that an amplitude value of the electromagnetic wave (110) changes by less than 5 % between the at least three receivers (115).

**7.** Apparatus as claimed in any of claims 3 to 6, wherein the predetermined wavelength ($\lambda$) ranges from 10 cm to 200 cm or from 40 to 120 cm.

**8.** Apparatus as claimed in any of the previous claims, wherein the evaluation unit (120) is configured to determine a relative height difference ($t_1$, $t_2$) between the at least three receivers in that the transmitter (105) may be positioned in the vicinity of one of the three receivers (115), and that the relative height differences ($t_1$, $t_2$) of the three receivers (115) may be determined on the basis of runtime or phase differences.

**9.** Apparatus as claimed in any of the previous claims, wherein the transmitter (105) is mounted on a blood pressure meter.

**10.** Apparatus as claimed in any of the previous claims, wherein the transmitter (105) may be mounted on a wrist.

**11.** Apparatus as claimed in any of the previous claims, comprising a first receiver (115a), a second receiver (115b) and a third receiver (115c), and wherein the first, second and third receivers (115a, 115b, 115c) may be mounted on a human body, so that the first receiver (115a) may be mounted at the level of a shoulder, the second receiver (115b) may be mounted at heart level, and the third receiver (115c) may be mounted at waist level.

**12.** Apparatus as claimed in any of the previous claims, further comprising a fourth receiver, said fourth receiver being configured to receive the signal (110), and the evaluation unit (120) being adapted to utilize the received signal of the fourth receiver as a control measurement so as to determine an error rate.

**13.** Method for performing a height correction of a measured blood pressure at a measuring position of a living being, comprising:

sending out a signal (110) by a transmitter (105) from near a measuring position;
receiving the signal (110) by at least three receivers (115), said at least three receivers (115) being mounted at locations at different heights of the living being;
and
correcting the measured blood pressure on the basis of runtime or phase differences of the signal received at the at least three receivers (115).

**14.** Method as claimed in claim 13, further comprising measuring of relative height differences ($t_1$, $t_2$).

**15.** Method as claimed in claim 13, further comprising positioning the transmitter (105) at a second receiver (115b) and determining relative height differences ($t_1$, $t_2$) from the runtime or phase differences.

**Revendications**

**1.** Dispositif de correction de hauteur d'une pression sanguine mesurée à un endroit de mesure d'un être vivant, aux caractéristiques suivantes:

un émetteur (105) destiné à émettre un signal (110) à partir d'une proximité d'un endroit de mesure;
au moins trois récepteurs (115) destinés à recevoir le signal (110), les récepteurs (115) pouvant être placés à des endroits de hauteur différente de l'être vivant; et
une unité d'évaluation (120) destinée à corriger la pression sanguine mesurée sur base de différences de temps de propagation ou de phase des signaux reçus aux au moins trois récepteurs (115).

**2.** Dispositif selon la revendication 1, dans lequel l'unité d'évaluation (120) est conçue pour déterminer, à partir des différences de temps de propagation ou de phase, une différence de hauteur (125) de l'émetteur (105) par rapport à un premier récepteur (115a).

**3.** Dispositif selon la revendication 1 ou la revendication 2, dans lequel le signal présente une onde électromagnétique (110) de longueur d'onde prédéterminée ($\lambda$) et dans lequel les au moins trois récepteurs (115) sont conçus pour transmettre des signaux de réception (117) à l'unité d'évaluation (120).

**4.** Dispositif selon la revendication 3, dans lequel les récepteurs (115) captent les phases de l'onde électromagnétique (110) reçue et les signaux de réception (117) présentent des informations sur les valeurs de phase reçues, et dans lequel l'unité d'évaluation présente un premier discriminateur de phases (122) destiné à déterminer une première différence de phase $\Delta\varphi_1$ entre une deuxième valeur de phase reçue et une première valeur de phase reçue; et un deuxième discriminateur de phases (124) destiné à déterminer une deuxième différence de phase $\Delta\varphi_2$ entre la troisième valeur de phase reçue et la deuxième valeur de phase reçue.

**5.** Dispositif selon l'une de la revendication 3 ou de la revendication 4, dans lequel la longueur d'onde prédéterminée ($\lambda$) correspond au moins au double de la longueur de bras d'un être humain.

**6.** Dispositif selon l'une des revendications 3 à 5, dans lequel la longueur d'onde prédéterminée ($\lambda$) se situe au-dessous d'une longueur d'onde maximale et la longueur d'onde maximale est donnée par le fait qu'une valeur d'amplitude de l'onde électromagnétique (110) entre les au moins trois récepteurs (115) varie de moins de 5 %.

**7.** Dispositif selon l'une des revendications 3 à 6, dans lequel la longueur d'onde prédéterminée ($\lambda$) se situe dans une plage comprise entre 10 cm et 200 cm ou dans une plage comprise entre 40 et 120 cm.

**8.** Dispositif selon l'une des revendications précédentes, dans lequel l'unité d'évaluation (120) est conçue pour déterminer une différence de hauteur relative ($t_1$, $t_2$) entre les au moins trois récepteurs par le fait que l'émetteur (105) peut être positionné à proximité de l'un des trois récepteurs (115) et que sur base des différences de temps de propagation ou de phase peuvent être déterminées les différences de hauteur relatives ($t_1$, $t_2$) des trois récepteurs (115).

9. Dispositif selon l'une des revendications précédentes, dans lequel l'émetteur (105) est placé sur un appareil de mesure de pression sanguine.

10. Dispositif selon l'une des revendications précédentes, dans lequel l'émetteur (105) peut être placé sur un poignet.

11. Dispositif selon l'une des revendications précédentes, présentant un premier récepteur (115a), un deuxième récepteur (115b) et un troisième récepteur (115c) et le premier, le deuxième et le troisième récepteur (115a, 115b, 115c) pouvant être placés sur le corps humain, de sorte que le premier récepteur (115a) puisse être placé à hauteur d'une épaule, le deuxième récepteur (115b) à hauteur du coeur et le troisième récepteur (115c) à hauteur de la taille.

12. Dispositif selon l'une des revendications précédentes, présentant par ailleurs un quatrième récepteur et le quatrième récepteur étant conçu pour recevoir le signal (110) et l'unité d'évaluation (120) étant conçue pour utiliser le signal reçu du quatrième récepteur comme mesure de contrôle, pour déterminer un taux d'erreur.

13. Procédé de correction de hauteur d'une pression sanguine mesurée à un endroit de mesure d'un être vivant, aux étapes suivantes consistant à:

envoyer un signal (110) par un émetteur (105) à partir d'une proximité d'un endroit de mesure;
recevoir le signal (110) par au moins trois récepteurs (115), les au moins trois récepteurs (115) étant placés à des endroits de hauteur différente de l'être vivant; et
corriger la pression sanguine mesurée sur base des différences de temps de propagation ou de phase du signal reçu aux au moins trois récepteurs (115).

14. Procédé selon la revendication 13, comprenant par ailleurs une mesure de différences de hauteur relatives ($t_1$, $t_2$).

15. Procédé selon la revendication 13, comprenant par ailleurs un positionnement de l'émetteur (105) à un deuxième émetteur (115b) et une détermination de différences de hauteur relatives ($t_1$, $t_2$) à partir des différences de temps de propagation ou de phase.

Fig. 1

Fig. 2

Fig. 3a

Fig. 3b

**EP 2 150 172 B1**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19757974 A